# EUROPEAN PATENT APPLICATION

(11) **EP 4 603 504 A1**
(43) Date of publication of application: **20.08.2025**
(21) Application number: 24157666.9
(22) Date of filing: 14.02.2024
(51) Int. Cl.: C07K 7/06, C07K 14/81, A61K 47/68

(54) **NOVEL CATHEPSIN K AND L INHIBITORS**

(71) Applicant: ECOLE POLYTECHNIQUE FEDERALE DE LAUSANNE (EPFL), 1015 Lausanne (CH)
(72) Inventor: The designation of the inventor has not yet been filed
(74) Representative: Vossius & Partner Patentanwälte Rechtsanwälte mbB

(57) **Abstract**

The present invention relates to a cathepsin K inhibitor comprising or consisting of Formula (I) (X₁)(X₂)(X₃)(X₄)(Y)(X₅)(X₆)(X₇) Formula (I), wherein (X₁) is the amino acid K; (X₂) is an amino acid selected from R and W, and is preferably R; (X₃) is an amino acid selected from R and W, and is preferably R; (X₄) is the amino acid L; (X₅) is an amino acid selected from Y, V and W, and is preferably V or Y, and is most preferably V; (X₆) is an amino acid selected from I, M, L, W and R, and is preferably I, L or W; and is most preferably I; (X₇) is an amino acid selected from H, W, A T, R; and is preferably H or R, and is most preferably R; and (Y) is a Michael acceptor. The present invention also relates to a cathepsin L inhibitor comprising or consisting of Formula (II) (X₁)(X₂)(X₃)(X₄)(Y)(X₅)(X₆)(X₇) Formula (II), wherein (X₁) is the amino acid K; (X₂) is the amino acid R; (X₃) is an amino acid selected from R, L and M, and is preferably L; (X₄) is an amino acid selected from L, W, F and Y, and is preferably W; (X₅) is an amino acid selected from V, H and R, and is preferably V; (X₆) is an amino acid selected from M, L, I, W and R, and is preferably L; (X₇) is an amino acid selected from W, A T, R; and is preferably W; and (Y) is a Michael acceptor.

## Description

The present invention relates to a cathepsin K inhibitor comprising or consisting of Formula (I)

(X₁)(X₂)(X₃)(X₄)(Y)(X₅)(X₆)(X₇) Formula (I)

wherein (X₁) is the amino acid K; (Xz) is an amino acid selected from R and W, and is preferably R; (Xs) is an amino acid selected from R and W, and is preferably R; (X₄) is the amino acid L; (X₅) is an amino acid selected from Y, V and W, and is preferably V or Y, and is most preferably V; (Xe) is an amino acid selected from I, M, L, W and R, and is preferably I, L or W; and is most preferably I; (X₇) is an amino acid selected from H, W, A T, R; and is preferably H or R, and is most preferably R; and (Y) is a Michael acceptor. The present invention also relates to a cathepsin L inhibitor comprising or consisting of Formula (II)

(X₁)(X₂)(X₃)(X₄)(Y)(X₅)(X₆)(X₇) Formula (II)

wherein (X₁) is the amino acid K; (X₂) is the amino acid R; (Xs) is an amino acid selected from R, L and M, and is preferably L; (X₄) is an amino acid selected from L, W, F and Y, and is preferably W; (X₅) is an amino acid selected from V, H and R, and is preferably V; (Xe) is an amino acid selected from M, L, I, Wand R, and is preferably L; (X₇) is an amino acid selected from W, A T, R; and is preferably W; and (Y) is a Michael acceptor.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

In the past years, functional annotations of cancer genomic lesions have steered cancer treatment towards more targeted and personalized therapies. This approach in combination with the introduction of immunotherapies has significantly improved cancer treatment. However, curative approaches are still rare and resistance to treatments and disease relapse are frequent and linked with tumor evolution.

Cathepsin proteins were identified as an interesting therapeutic target in cancer and immunological diseases (see for example, Wilkinson et al. (2016), Biological Chemistry https://doi.org/10.1515/hsz-2015-0114).

Cathepsin K is known as a versatile potential biomarker and therapeutic target for various Cancers (Qian et al. (2022), Curr Oncol. 2022 Aug; 29(8): 5963-5987). Upregulation of cathepsin L plays an important role in angiogenesis and metastasis through its ability to degrade the extracellular matrix, facilitating tissue remodeling and tumour cell invasion. Cathepsin L is therefore also a potential therapeutic target for anticancer medicine (Tabish et al. (2020), Front. Nanotechnol., Volume 2 - 2020 | https://doi.org/10.3389/fnano.2020.00001).

In general, cathepsin proteins are important for the regulation of antigen presentation and adaptive immune response, and inhibition of cathepsin proteins could be beneficial in genetic auto-immune diseases.

Hence, in order to further advance the therapeutic options for treating and preventing cancer and immunological diseases cathepsin K and L inhibitors are needed. Such cathepsin inhibitors can be used alone or in combination with immunotherapies for the treatment and the prevention of cancer and immunological diseases.

Thus, the present invention relates in a first aspect to a cathepsin K inhibitor comprising or consisting of Formula (I)

(X₁)(X₂)(X₃)(X₄)(Y)(X₅)(X₆)(X₇) Formula (I)

wherein (X₁) is the amino acid K; (Xz) is an amino acid selected from R and W, and is preferably R; (Xs) is an amino acid selected from R and W, and is preferably R; (X₄) is the amino acid L; (X₅) is an amino acid selected from Y, V and W, and is preferably V or Y, and is most preferably V; (Xe) is an amino acid selected from I, M, L, W and R, and is preferably I, L or W; and is most preferably I; (X₇) is an amino acid selected from H, W, A T, R; and is preferably H or R, and is most preferably R; and (Y) is a Michael acceptor.

Cathepsins are protease enzymes, categorized into multiple families. They can be a serine protease, cysteine protease, or aspartyl protease. There are currently about 15 classes of cathepsins in humans (Patel et a. (2018), Biomed Pharmacother. 2018 Sep; 105: 526-532). These enzymes are active in the low pH milieu of lysosomes and are versatile in their functions. Like other enzymes, they are vital for the normal physiological functions such as digestion, blood coagulation, bone resorption, ion channel activity, innate immunity, complement activation, apoptosis, vesicular trafficking, autophagy, angiogenesis, and proliferation among scores of others. Numerous pathologies have been attributed to the dysregulated cathepsins, some of which include arthritis, periodontitis, pancreatitis, macular degeneration, muscular dystrophy, atherosclerosis, obesity, stroke, Alzheimer's disease, schizophrenia, tuberculosis, and Ebola.

Cathepsin K (CTSK) is a cysteine protease and known to be involved in cancer. Cathepsin K is a member of the peptidase C1 protein family, is expressed predominantly in osteoclasts. Cathepsin K is a protease, which is defined by its high specificity for kinins, that is involved in bone resorption. The enzyme's ability to catabolize elastin, collagen, and gelatin allows it to break down bone and cartilage. This catabolic activity is also partially responsible for the loss of lung elasticity and recoil in emphysema.

Cathepsin K inhibitors show great potential in the treatment of osteoporosis. Cathepsin K is degraded by Cathepsin S, in a process referred to as Controlled Cathepsin Cannibalism. Cathepsin K is expressed in a significant fraction of human breast cancers, where it could contribute to tumor invasiveness. Cathepsin K has also been found to be over-expressed in glioblastoma.

The second aspect of the invention described in greater detail herein below relates to a cathepsin L inhibitor. Cathepsin L (CTSL) is a cysteine protease and known to be involved in cancer. Cathepsin L belongs to the papain subfamily of cysteine proteases. Cathepsin L (CTSL) is a lysosomal protease and is involved in a wide range of cellular functions with implications in disease. Many studies have shown the involvement of CTSL in cancer. For example, nuclear CTSL is shown to be up-regulated in breast cancer. Inhibition of CTSL can increase the therapeutic efficacy of radiation. In both breast and prostate cancer cells, up-regulation of CTSL leads to increased tumor cell invasion. Elevation of CTSL serum levels in ovarian cancer patients is correlated with malignancy and metastasis to the lymph nodes, and in epithelial ovarian cancer cells, up-regulation of CTSL also leads to increased migration and tubule formation. Additionally, CTSL-mediated degradation of the extracellular matrix leads to metastasis and lymphatic invasion in pancreatic cancer- CTSL expression is also increased in pancreatic tumor tissue.

The term "cathepsin inhibitor" refers to agents being able to inhibit the protease activity of cathepsin, in particular cathepsin K or L. Inhibition of Cathepsin K or L can be assessed for example by measuring the enzymatic transformation of Cathepsin K or L substrates (peptides of 9-10 amino acids) into products (peptides of 4-6 amino acids), for example, by a FRET assay as described in the examples. Inhibition of Cathepsin K or L can be also assessed at the functional level, for example, for cathepsin K assays can be performed to measure bone resorption.

The cathepsin inhibitor of the first and second aspect comprises the 7 amino acids (X₁) to (X₇) and therefore may comprise further amino acids at the "N-terminus" (i.e. next to (X₁)) and/or C-terminus (i.e. next to (X₇)). The total number of amino acids in the cathepsin inhibitor is with increasing preference 50 or less, 40 or less, 30 or less, 20 or less, 15 or less, 10 or less, 9 or less, 8 or less and most preferably 7. In the most preferred case no amino acids otherthan (X₁) to (X₇) are present in the cathepsin inhibitor.

The cathepsin inhibitor of the first and second aspect might be linear or circular and is preferably linear. In case of a circular cathepsin inhibitor the circle is preferably formed by connecting the side chains of two amino acids either directly (e.g. via disulfide bonds in the case of two cysteine) or via a connecting molecule. It is in particular to form a circle between the side chains of (X₁) and (X₇) or two amino acids that can be found at the "N-terminus" (i.e. next to (X₁)) and C-terminus (i.e. next to (X₇)) of the cathepsin inhibitor.

The term "connecting molecule" as used herein refers to a molecule which is capable of connecting the side chains of at least two amino acids via covalent bonds whereby a ring or cycle is formed. A covalent bond is a chemical bond that involves the sharing of electron pairs between atoms. For this purpose the connecting molecule comprises at least two functional groups. A functional group designates a specific group of atoms or bonds within the connecting molecule that is responsible for connecting the side chain of at least two amino acids to the connecting molecule. For this purpose the functional group in general undergoes a chemical reaction with at least one atom in the side chain of the amino acid. The chemical reaction results in a covalent bond between the functional group and the side chain. Non-limiting examples of functional groups are alkyl, alkenyl, alkynyl, phenyl, benzyl, halo (such as fluoro or chloro), hydroxyl, carbonyl, aldehyde, haloformyl, carbonate ester, carboxylate, carboxyl, ester, methoxy, hydroperoxy, peroxy, ether, hemiacetal, hemiketal, acetal, ketal, orthoester, heterocycle, orthocarbonate ester, carboxamide, amine (including primary, secondary and tertiary amine), imine, azide, azo compound, (iso)cyanate, nitrate, nitrite, sufhydryl, sulfide, disulfide, sulfinyl, sulfonyl, sulfin, sulfo, (iso)thiocyanate, carbonothiol, carbonothioyl, phosphino (such as phospate), borono, boronate, boroniate and borino. The at least two functional groups of the connecting molecule may be the same or different and are preferably the same.

The term "amino acid" as used herein refers to an organic compound composed of amine (-NH₂ or -NH) and carboxylic acid (-COOH) functional groups, generally along with a side-chain specific to each amino acid. The simplest amino acid glycine does not have a side chain (formula H₂NCH₂COOH). In amino acids that have a carbon chain attached to the α-carbon (such as lysine), the carbons are labelled in the order α, β, γ, δ, and so on. In some amino acids, the amine group may be attached, for instance, to the α-, β- or γ-carbon, and these are therefore referred to as α-, β- or γ-amino acids, respectively. The term "amino acid" preferably describes α-amino acids (also designated 2-, or alpha-amino acids) which generally have the generic formula H₂NCHRCOOH, wherein R is an organic substituent being designated "side-chain", and also beta-, gamma- and delta-amino acids, that contain multiple carbon atoms between the amine and the carboxylic acid. In the simplest α-amino acid alanine (formula: H₂NCHCH₃COOH) the side chain is a methyl group. The amino acids in accordance with the present invention are L-amino acids or D-amino acids.

The amino acids comprise the so-called standard or canonical amino acids. These 21 α-amino acids are encoded directly by the codons of the universal genetic code. They are the proteinogenic α-amino acids found in eukaryotes. These amino acids are referred to herein in the so-called one-letter code:

| | | | |
|---|---|---|---|
| **G** | Glycine | **P** | Proline |
| **A** | Alanine | **V** | Valine |
| **L** | Leucine | **I** | Isoleucine |
| **M** | Methionine | **C** | Cysteine |
| **F** | Phenylalanine | **Y** | Tyrosine |
| **W** | Tryptophan | **H** | Histidine |
| **K** | Lysine | **R** | Arginine |
| **Q** | Glutamine | **N** | Asparagine |
| **E** | Glutamic Acid | **D** | Aspartic Acid |
| **S** | Serine | **T** | Threonine |
| **U** | Selenocysteine | | |

The four amino acids (X₁)(X₂)(X₃)(X₄) as well as the three amino acids (X₅)(X₆)(X₇) within the inhibitor of the invention are each connected to form a "peptide". A peptide generally designates a short chain of amino acids linked by peptide bonds. The term "peptide" as used herein, thus, designates short chains of amino acids that are preferably linked by peptide bonds.

The cathepsin inhibitor of the first and second aspect of the invention is not only composed of amino acids being connected to form a peptide but comprises in addition (Y), which is a Michael acceptor. Michael additions (or Michael reactions) are C-C bond formation reactions in which nucleophilic addition of a carbanion or another nucleophile to an α,β-unsaturated carbonyl compound containing an electron withdrawing group occurs. In this scheme, R and R' on the nucleophile (the Michael donor) represent electron-withdrawing substituents such as acyl and cyano groups, which make the adjacent methylene hydrogen sufficiently acidic to form a carbanion when reacted with the base, B: as shown in the below reaction. The R" substituent is bound to the activated alkene, also called a Michael acceptor. The nature of the Michael acceptor will be further detailed herein below.

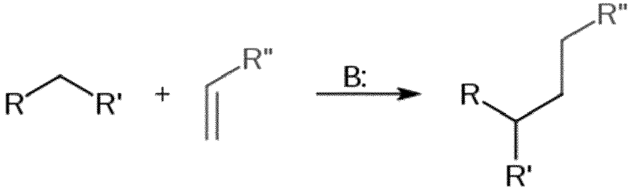

Since the cathepsin inhibitor of the first and second aspect of the invention is composed of seven amino acids and the Michael acceptor as illustrated by formula (I) and formula (II), it is to be understood that the cathepsin inhibitor of the invention does not occur in nature but has to be chemically synthesized as also illustrated by the appended examples. The cathepsin inhibitor also cannot be encoded by a nucleic acid molecule, such as an expression vector.

The inventors of the present invention previously designed cathepsin S inhibitors (WO 2023/021190). The cathepsin S inhibitors were designed based on the knowledge that CD74 is a natural substrate of cathepsin S and the computational analysis of the interaction site between CD74 and cathepsin S. After the amino acids of CD74 that interact with cathepsin S were identified several candidate cathepsin inhibitors were produced by adding a Michael acceptor at different position.

By a mutagenesis screen it was surprisingly possible to change the specificity of the cathepsin S inhibitor from cathepsin S to cathepsin K and L. Initially the cathepsin K and L inhibitor NNPI-E15 was found that inhibits both cathepsin K and L.

By a further mutagenesis screen it was then possible to increase the specificity to cathepsin K. The best and highly effective cathepsin K inhibitor is NNPI-F17.

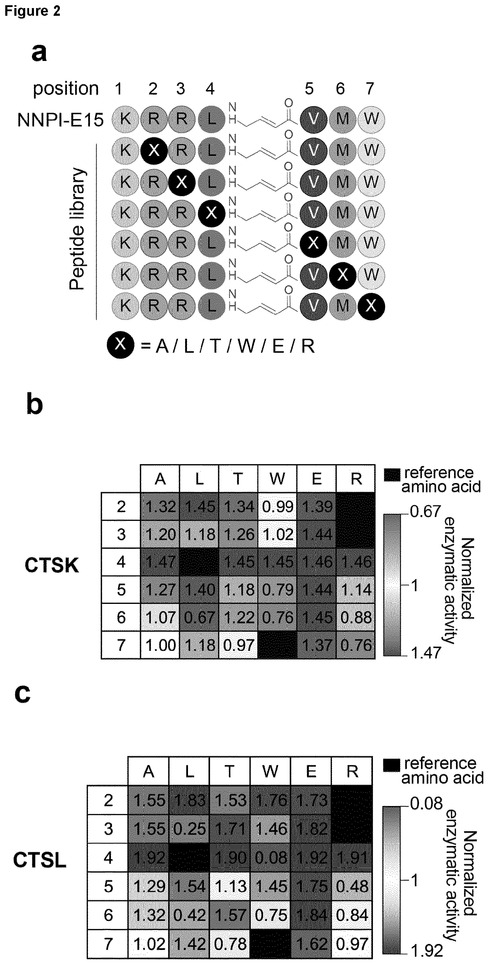

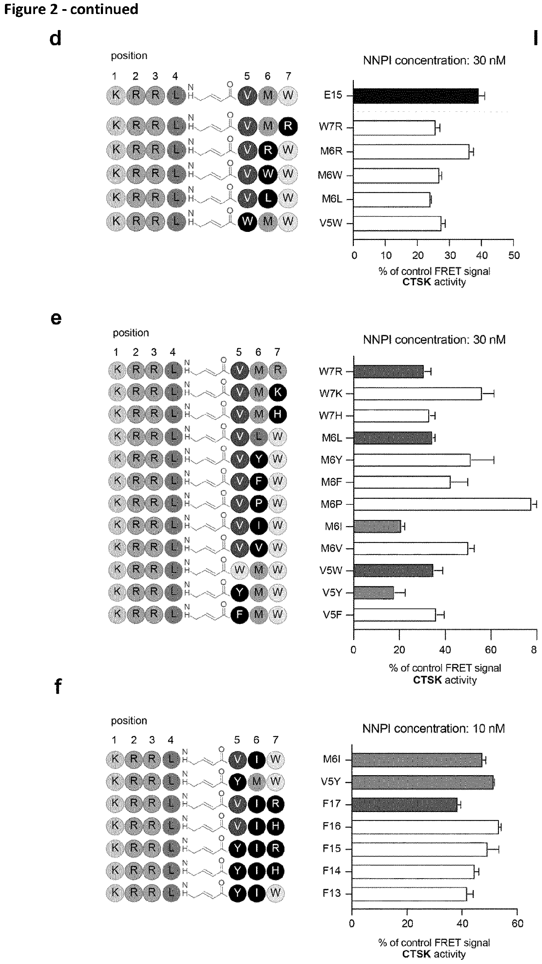

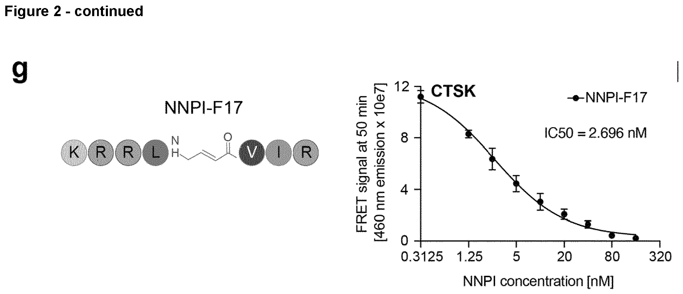

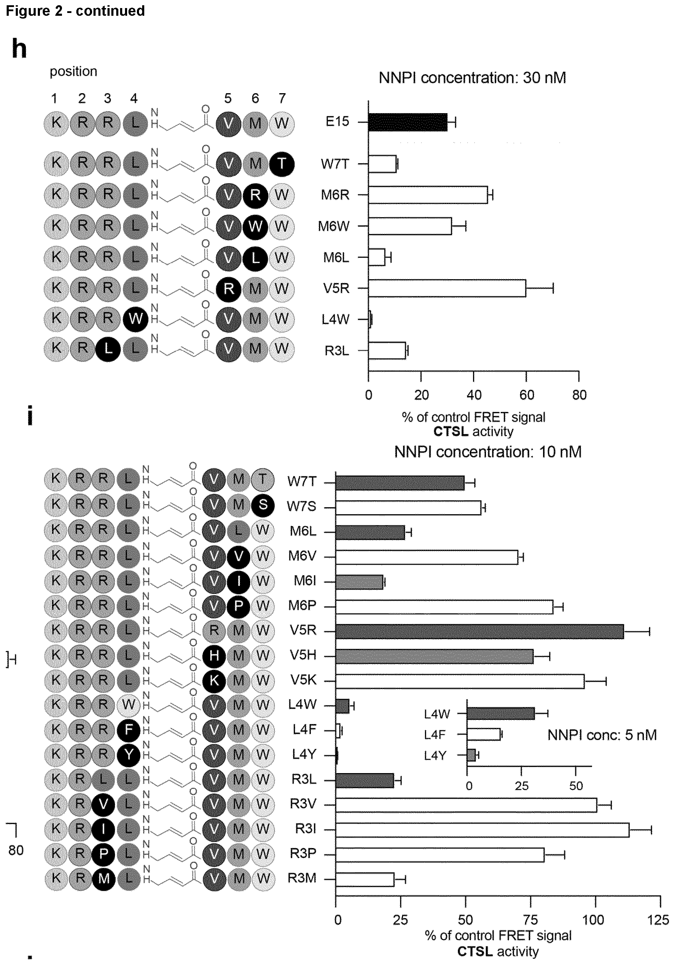

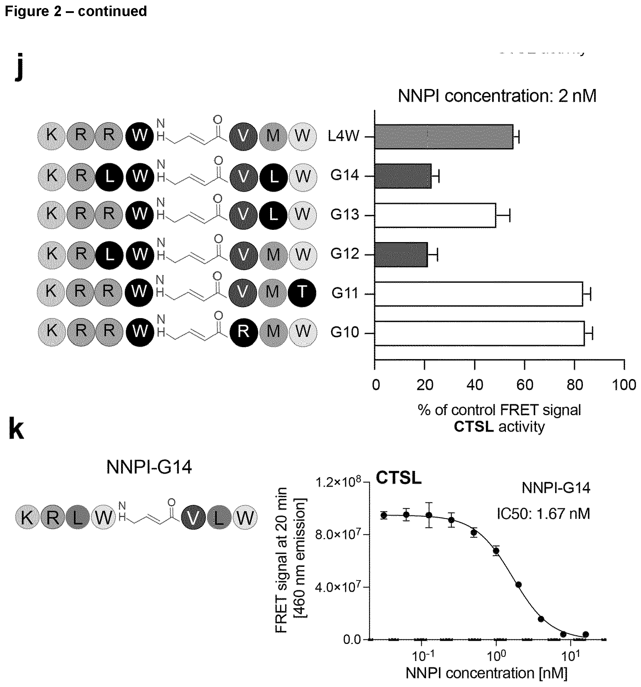

The structure of formula (I) comprises for amino acid positions (X₁) to (X₇) inhibitor NNPI-E15 and amino acid changes as show in Fig 2 b and d-f that either improved or did not diminish the inhibitory capacity for cathepsin K.

The structure of formula (I) is therefore a Markush formula of very potent cathepsin K inhibitors. To the best knowledge of the inventors the structure of formula (I), let alone its cathepsin K inhibitory capacity is not known from the prior art.

The preferred options for amino acids (X₁) to (X₇) improve CTSK inhibition. The most preferred amino acids (X₁) to (X₇) in the structure of formula (I) are those amino acids of the best cathepsin K inhibitor NNPI-F17 as shown in Fig 2g.

In accordance with a preferred embodiment the cathepsin K inhibitor comprises or consists of KRRL(Y)VIR, KRRL(Y)VMR, KRRL(Y)VRW, KRRL(Y)VWW, KRRL(Y)VLW, KRRL(Y)WMW, KRRL(Y)VIW, KRRL(Y)YMW, KRRL(Y)VMH, KRRL(Y)VIH, KRRL(Y)YIR or KRRL(Y)YIH or KRRL(Y)YIW.

The above preferred embodiment is directed to all cathepsin K inhibitors in Fig 2b, d, e, f and g that inhibit cathepsin K even better than NNPI-E15.

In accordance with a more preferred embodiment the cathepsin K inhibitor comprises or consists of KRRL(Y)VIR, KRRL(Y)VIW, KRRL(Y)YMW, KRRL(Y)VMH, KRRL(Y)VIH, KRRL(Y)YIR, KRRL(Y)YIH or KRRL(Y)YIW.

The above more preferred embodiment is directed to all cathepsin K inhibitors in Fig 2f. These cathepsin K inhibitors are the most preferred cathepsin K inhibitor NNPI-F17 as well as the cathepsin K inhibitors that perform similarly good as NNPI-F17.

In accordance with an even more preferred embodiment the cathepsin K inhibitor comprises or consists of KRRL(Y)VIR.

KRRL(Y)VIR is the most preferred cathepsin K inhibitor NNPI-F17 as shown in Fig 2g.

The present invention relates in a second aspect to a cathepsin L inhibitor comprising or consisting of Formula (II)

(X₁)(X₂)(X₃)(X₄)(Y)(X₅)(X₆)(X₇) Formula (II)

wherein (X₁) is the amino acid K; (X₂) is the amino acid R; (Xs) is an amino acid selected from R, L and M, and is preferably L; (X₄) is an amino acid selected from L, W, F and Y, and is preferably W; (X₅) is an amino acid selected from V, H and R, and is preferably V; (Xe) is an amino acid selected from M, L, I, Wand R, and is preferably L; (X₇) is an amino acid selected from W, A T, R; and is preferably W; and (Y) is a Michael acceptor.

The above definitions and preferred embodiments as described in connection with the first aspect of the invention apply mutatis mutandis to the second aspect of the invention.

As discussed above initially the cathepsin K and L inhibitor NNPI-E15 was found that inhibits both cathepsin K and L.

By a further mutagenesis screen it was then possible to increase the specificity to cathepsin L. The best and highly effective cathepsin L inhibitor is NNPI-G14.

The structure of formula (II) comprises for amino acid positions (X₁) to (X₇) inhibitor NNPI-E15 and amino acid changes as show in Fig 2 c and h-j that either improved or did not diminish the inhibitory capacity for cathepsin L.

The structure of formula (II) is therefore a Markush formula of very potent cathepsin L inhibitors. To the best knowledge of the inventors the structure of formula (II), let alone its cathepsin L inhibitory capacity is not known from the prior art.

The preferred options for amino acids (X₁) to (X₇) improve CTSL inhibition. The most preferred amino acids (X₁) to (X₇) in the structure of formula (II) are those amino acids of the best cathepsin L inhibitor NNPI-G14 as shown in Fig 2k.

In accordance with a preferred embodiment the cathepsin L inhibitor comprises or consists of KRLW(Y)VLW, KRRW(Y)VMW, KRRW(Y)VLW, KRLW(Y)VMW, KRRF(Y)VMW, or KRRY(Y)VMW.

The above more preferred embodiment is directed to all cathepsin L inhibitors in Fig 2h to j that perform at least similarly good as NNPI-L4W in Fig 2. The cathepsin K inhibitors NNPI-L4W is already a significantly more potent cathepsin L inhibitor than the initial prototype inhibitor NNPI-E15.

Within the above preferred embodiment KRLW(Y)VLW and KRLW(Y)VMW are preferred since they are the two best cathepsin L inhibitors NNPI-G14 and NNPI-G12 as shown in Fig 2i.

In accordance with a more preferred embodiment the cathepsin L inhibitor comprises or consists of KRLW(Y)VLW.

KRLW(Y)VLW is the most preferred cathepsin L inhibitor NNPI-G14 as shown in Fig 2k.

In accordance with a preferred embodiment the Michael acceptor is an enone, a nitro group or a sulfonyl fluoride and is preferably an enone.

The Michael acceptor is usually a ketone, which makes the compound an enone, but it can also be a nitro group or a sulfonyl fluoride. An enone is a type of an α,β-unsaturated carbonyl that consists of an alkene conjugated to a ketone. The simplest enone is methyl vinyl ketone (butenone, CH2=CHCOCH3). Enones are typically produced using an aldol condensation or Knoevenagel condensation. Some commercially significant enones produced by condensations of acetone are mesityl oxide (dimer of acetone) and phorone and isophorone (trimers).

In accordance with a more preferred embodiment the Michael acceptor has the general formula (III) or (IV) 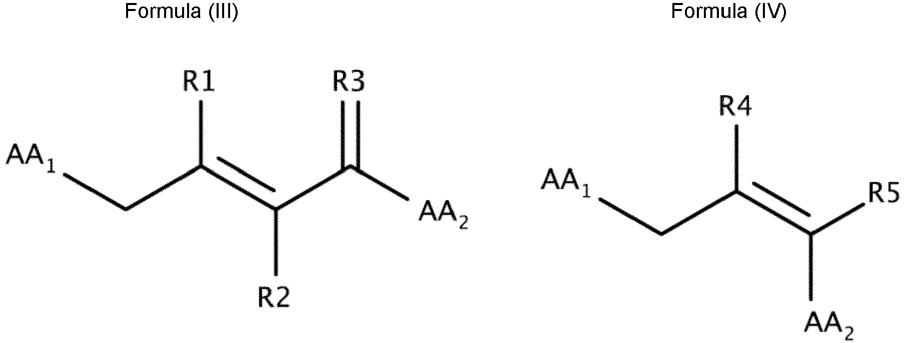 wherein
R1, R2 and R4 each independently is H or a halogen, wherein the halogen is preferably F;
R3 is chalcogen and is preferably O or S; and

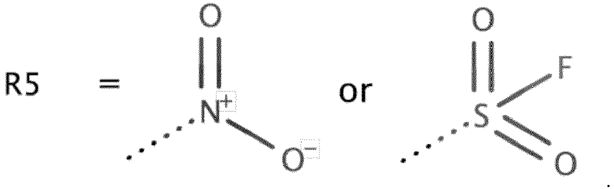

The compound of formula (III) is an enone. The compound of formula (IV) according to the first option of R5 is a nitro group, and the compound of formula (IV) according to the second option of R5 is a sulfonyl fluoride. The enone is again most preferred.

The halogen is preferably selected from fluorine (F), chlorine (CI), bromine (Br) and iodine (I), more preferably from F, CI and Br, even more preferably from F and Cl and is most preferably F.

A chalcogen is a chemical element in group 16 of the periodic table. The halogen is preferably selected from oxygen (O), sulfur (S), selenium (Se) and tellurium (Te), more preferably from O, S and Se, even more preferably from O and S and is most preferably O.

In accordance with a preferred embodiment the cathepsin inhibitor inhibits cathepsin K or L with IC₅₀ of below 100 nM, preferably below 50nM and most preferably below 10nM.

The IC50 values of the cathepsin (K as well as L) inhibitor of the invention is with increasing preference below 50µM, below 100 nM, 90 nM, 80 nM, 70 nM, 60 nM, 50 nM, 40 nM, 30 nM, 20 nM, and 10 nM. In this respect it is of note that the cathepsin K inhibitor NPPI-F17 displays an IC₅₀ of 2.7 nM for CTSK and the cathepsin L inhibitor NPPI-G14 displays an IC₅₀ of 1.67 nM for CTSL (Fig 2g,k).

In accordance with another preferred embodiment the cathepsin inhibitor comprises at its N- or C-terminus, preferably at its C-terminus a linking moiety.

A linking moiety (or linker moiety) generally relates to a functional group that is covalently bound to the inhibitor of the invention and can serve to link, generally covalently link a further compound to the inhibitor of the invention. Examples of such heterologous compounds will be provided herein below.

In accordance with a more preferred embodiment the linking moiety is an azide, alkyne phenol, secondary or tertiary amine, hydroxyl group, carbamate, or carbonate group, and is preferably an azide.

Non-limiting examples of suitable linking moieties are an azide, alkyne phenol, secondary or tertiary amine, hydroxyl group, carbamate. An azide is preferred since its it used in the examples in order to covalently link the inhibitor of the invention to an antibody.

A linking moiety, such as an alkyne or azide may be used in click-chemistry reactions. "Click-chemistry" is an art-established term; see e.g. Kolb et al. (2001) Click chemistry: diverse chemical function from a few good reactions. Angew. Chem. Int. Ed. 40 (11):2004; Sletten et al. (2009) Bioorthogonal Chemistry: Fishing for Selectivity in a Sea of Functionality. Angew. Chem. Int. Ed.48:6998; Jewett et al. (2010) Cu-free click cycloaddition reactions in chemical biology. Chem. Soc. Rev. 39(4):1272; Best et al. (2009) Click Chemistry and Bioorthogonal Reactions: Unprecedented Selectivity in the Labeling of Biological Molecules. Biochemistry.48:6571; and Lallana et al. (2011) Reliable and Efficient Procedures for the Conjugation of Biomolecules through Huisgen Azide-Alkyne Cycloadditions. Angew. Chem. Int. Ed. 50:8794. While there are a number of reactions that fulfill the criteria, the Huisgen 1,3-dipolar cycloaddition of azides and terminal alkynes has emerged as the frontrunner and are also preferred in connection with the present invention.

The present invention relates in a third aspect to a fusion construct, wherein the cathepsin inhibitor of the first or second aspect of the invention is fused to a heterologous compound, preferably fused to a pharmaceutically and/or diagnostically active compound.

The definitions and preferred embodiments of the first and second aspect of the invention apply *mutatis mutandis* to the third aspect of the invention as far as being amenable with this third aspect.

A "fusion construct" as used herein defines the fusion of the inhibitor of the first or second aspect of the invention to a heterologous compound. The heterologous compound is not particularly limited. It can be a proteinaceous compound or a non-proteinaceous compound. Examples of a proteinaceous and non-proteinaceous compounds will be provided herein below. The compound may be selected from the group consisting of a pharmaceutically active compound and/or a diagnostically active compound. A pharmaceutically active compound is capable of exerting a curative or disease preventive effect *in vivo* within a subject and a diagnostically active compound is useful for diagnosing a disease *in vivo* within a subject or *in vitro* within a sample obtained from the subject.

In the case the compound is a proteinaceous compound (e.g. a cytokine or chemokine as described herein below), the fusion construct is also a fusion protein as defined herein above. In other words, the term "fusion constructs" comprises fusion proteins. The compound may either be directly fused to the inhibitor or via a linker. The linker is preferably selected from the group consisting of alkyl with 1 to 30 carbon atoms, polyethylene glycol with 1 to 20 ethylene moieties, polyalanine with 1 to 20 residues, caproic acid, substituted or unsubstituted poly-p-phenylene and triazol.

In a further preferred embodiment, said linker is selected from the group consisting of amino-n-alkyl, mercapto-n-alkyl, amino-n-alkyl-X-alkyl, mercapto-n-alkyl-X-alkyl, wherein X is selected from the group consisting of O, S-S and SO₂ and wherein the alkyl groups independently from each other have from 1 to 30 carbon atoms, preferably 3, 6 or 12 carbon atoms; or oligoethylenglycols having from one to about ten ethylenglycol moieties, preferably tri- or hexa-ethylenglycol. These and further suitable linkers are well known in the art and commercially available (see, for example, the catalogue from Glen Research, 22825 Davis Drive, Sterling, Virginia, 20164 USA). Further examples of linkers are the following: 5'-amino-modifiers (see e.g. B.A. Connolly and P. Rider, Nucleic Acids Res., 1985, 13, 4485; B.S. Sproat, B.S. Beijer, P. Rider, and P. Neuner, Nucleic Acids Res., 1987, 15, 4837; R. Zuckerman, D. Corey, and P. Shultz, Nucleic Acids Res., 1987, 15, 5305; P. Li, et al., Nucleic Acids Res., 1987, 15, 5275; G.B. Dreyer and P.B. Dervan, Proc. Natl. Acad. Sci. USA, 1985, 82, 968.); 5'-thiol-modifier C6 (see e.g. B.A. Connolly and P. Rider, Nucleic Acids Res., 1985, 13, 4485; B.S. Sproat, B.S. Beijer, P. Rider, and P. Neuner, Nucleic Acids Res., 1987, 15, 4837; R. Zuckerman, D. Corey, and P. Shultz, Nucleic Acids Res., 1987, 15, 5305; P. Li, et al., Nucleic Acids Res., 1987, 15, 5275.); 5'-thiol-modifier C6 S-S and thiol group at the 3'-terminus (see e.g. B.A. Connolly and R. Rider, Nucleic Acids Res., 1985, 13, 4485; B.A. Connolly, Nucleic Acids Res., 1987, 15, 3131-3139; N.D. Sinha and R.M. Cook, Nucleic Acids Res., 1988, 16, 2659; A. Kumar, S. Advani, H. Dawar, and G.P. Talwar, Nucleic Acids Res., 1991, 19, 4561; R. Zuckermann, D. Corey, and P. Schultz, Nucleic Acids Res., 1987, 15, 5305; K.C. Gupta, P. Sharma, S. Sathyanarayana, and P. Kumar, Tetrahedron Lett., 1990, 31, 2471-2474; U. Asseline, E. Bonfils, R. Kurfurst, M. Chassignol, V. Roig, and N.T. Thuong, Tetrahedron, 1992, 48, 1233-1254; Gregg Morin, Geron Corporation, Personal Communication.); spacer C3, spacer C12, and dspacer phosphoramidites (see e.g. M. Durard, K. Chevrie, M. Chassignol, N.T. Thuong, and J.C. Maurizot, Nucleic Acids Res., 1990, 18, 6353; M. Salunkhe, T.F. Wu, and R.L. Letsinger, J. Amer. Chem. Soc., 1992, 114, 8768-8772; N.G. Dolinnaya, M. Blumenfeld, I.N. Merenkova, T.S. Oretskaya, N.F.Krynetskaya, M.G. Ivanovskaya, M. Vasseur, and Z.A. Shabarova, Nucleic Acids Res., 1993, 21, 5403-5407; M. Takeshita, C.N. Chang, F. Johnson, S. Will, and A.P. Grollman, J. Biol. Chem., 1987, 262, 10171-10179; M.W. Kalnik, C.N. Chang, A.P. Grollman, and D.J. Patel, Biochemistry, 1988, 27, 924-931.); 3'-amino-modifier C7 CPG (see e.g. J.G. Zendegui, K.M. Vasquez, J.H. Tinsley, D.J. Kessler, and M.E. Hogan, Nucleic Acids Res., 1992, 20, 307.); 3'-Amino Photolabile C6 CPG (see e.g. D.J. Yoo and M.M. Greenberg, J. Org. Chem., 1995, 60, 3358-3364.; H. Venkatesan and M.M. Greenberg, J. Org. Chem., 1996, 61, 525-529; D.L. McMinn and M.M. Greenberg, Tetrahedron, 1996, 52, 3827-3840.

As discussed, in case the heterologous compound is a proteinaceous compound the fusion construct may also be designated "fusion protein". A "fusion protein" according to the present invention contains at least one additional heterologous amino acid sequence apart from the inhibitor of the first or second aspect of the invention. Often, but not necessarily, these additional sequences will be located at the Nor C-terminal end of the inhibitor of the invention. The amino acid sequence can either be directly or indirectly fused to the inhibitor of the invention. In case of an indirect fusion generally a peptide linker may be used for the fusion, such that a GS₍ₙ₎-linker, wherein n is 1 to 3.

Most preferably the inhibitor of the first or second aspect of the invention and the heterologous compound are fused via a linking moiety as described herein above.

In accordance with a preferred embodiment the heterologous compound is selected from the group consisting of:
(i) antibody or fragment thereof;
(ii) an antibody mimetic, preferably selected from the group consisting of Anticalins, Affibodies, Adnectins, DARPins, Avimers, Nanofitins, Affilins, β-Wrapins, ADAPT, Monobodies, Raslns, FingRs, Pronectins, Centyrins, Affimers, Adhirons, Affitins, αReps, Repebodies, i-bodies, Fynomers and Kunitz domain proteins;
(iil) a cytokine, preferably cytokines selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta;
(iv) a toxic compound, preferably a small organic compound or polypeptide, and preferably a toxic compound selected from the group consisting of calicheamicin, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin and recombinant gelonin;
(v) a chemokine, preferably a chemokine selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, Eotaxin, Eotaxin-2, I-309, MPIF-1, 6Ckine, CTACK, MEC, Lymphotactin and Fractalkine;
(vi) a fluorescent dye, preferably a component selected from a Alexa Fluor or Cy dye;
(viii) a photosensitizer, preferably bis(triethanolamine)Sn(IV) chlorine6 (SnChe6);
(viii) a pro-coagulant factor, preferably a tissue factor;
(ix) an enzyme, preferably an enzyme selected from the group consisting of carboxy-peptidases, glucuronidases and glucosidases;
(x) a radionuclide selected either from the group of gamma-emitting isotopes, preferably 99mTc, 123I, 111In, or from the group of positron emitters, preferably 18F, 64Cu, 68Ga, 86Y, 124I, or from the group of beta-emitters, preferably 1311, 90Y, 177Lu, 67Cu, or from the group of alpha-emitters, preferably 213Bi, 211At;
(xi) nanoparticles.

Among this list of fusion partners (i) an antibody or fragment thereof and (ii) an antibody mimetic are preferred and an antibody or fragment thereof is most preferred. These fusion partners are preferred since they can be designed to bind virtually any target of interest. This includes targets that are of diagnostic and therapeutic interest.

The term "antibody", also known as an immunoglobulin (Ig), as used in accordance with the present invention comprises, for example, polyclonal or monoclonal antibodies. Furthermore, comprised in the term "antibody" are multimeric formats, such as minibodies, diabodies, tribodies or triplebodies, or tetrabodies (see, for example, Harlow and Lane "Antibodies, A Laboratory Manual", Cold Spring Harbor Laboratory Press, 1998; Harlow and Lane "Using Antibodies: A Laboratory Manual" Cold Spring Harbor Laboratory Press, 1999; Altshuler EP, Serebryanaya DV, Katrukha AG. 2010, Biochemistry (Mosc)., vol. 75(13), 1584; Holliger P, Hudson PJ. 2005, Nat Biotechnol., vol. 23(9), 1126). The multimeric formats in particular comprise bispecific antibodies that can simultaneously bind to two different types of antigen. Non-limiting examples of bispecific antibodies formats are Biclonics (bispecific, full length human IgG antibodies), DART (Dual-affinity Re-targeting Antibody) and BiTE (consisting of two single-chain variable fragments (scFvs) of different antibodies) molecules (Kontermann and Brinkmann (2015), Drug Discovery Today, 20(7):838-847). The term "antibody" also includes embodiments such as chimeric (human constant domain, non-human variable domain), single chain and humanised (human antibody with the exception of non-human CDRs) antibodies.

In accordance with the present invention, antibody fragments comprise, *inter alia,* Fab or Fab' fragments, F(ab')₂, Fv or scFv fragments, single domain VH, VL orV-like domains, such as VhH or V-NAR-domains, as well as multimeric formats such as minibodies, diabodies, tribodies, triplebodies, tetrabodies or chemically conjugated Fab'-multimers (see, for example, Altshuler, E. et al. [2010] Biochem. (Mosc.) 75:1584-1605 or Holliger, P. & Hudson, P.J. [2005] Nat. Biotechnol. 23:1126-1136).

"Anticalins" are an emerging class of clinical-stage biopharmaceuticals with high potential as an alternative to antibodies. Anticalin molecules are generated by combinatorial design from natural lipocalins, which are abundant plasma proteins in humans, and reveal a simple, compact fold dominated by a central β-barrel, supporting four structurally variable loops that form a binding site. Reshaping of this loop region results in Anticalin proteins that can recognize and tightly bind a wide range of medically relevant targets, from small molecules to peptides and proteins, as validated by X-ray structural analysis. Their robust format allows for modification in several ways, both as fusion proteins and by chemical conjugation, for example, to tune plasma half-life (Rothe and Skerra (2018) BioDrugs 32, 233-243.).

"Affibodies", in accordance with the present invention, are a family of antibody mimetics derived from the Z-domain of staphylococcal protein A. Affibodies are structurally based on a three-helix bundle domain. An affibody has a molecular mass of around 6 kDa and is stable at high temperatures and under acidic or alkaline conditions. Target specificity is obtained by randomisation of amino acids located in two alpha-helices involved in the binding activity of the parent protein domain (Feldwisch, J & Tolmachev, V. [2012] Methods Mol. Biol. 899:103-126).

"Adnectins" and also "Monobodies", in accordance with the present invention, are based on the 10th extracellular domain of human fibronectin III (10Fn3), which adopts an Ig-like sandwich fold with 2 to 3 exposed loops, but lacks the central disulphide bridge (Gebauer, M. & Skerra, A. [2009] Curr. Opin. Chem. Biol. 13:245-255). Adnectins and Monobodies with the desired target specificity can be genetically engineered by introducing modifications into specific loops or other surface areas of the protein.

"DARPins", in accordance with the present invention, are designed ankyrin repeat domains that provide a rigid interface arising from typically three repeats corresponding to an artificial consensus sequence, whereby six positions per repeat are randomised. Consequently, DARPins lack structural flexibility (Gebauer, M. & Skerra, A. [2009] Curr. Opin. Chem. Biol. 13:245-255).

The term "Avimer", as used herein, refers to a class of antibody mimetics which consist of two or more peptide sequences of 30 to 35 amino acids each, which are derived from A-domains of various membrane receptors and which are connected by linker peptides. Binding of target molecules occurs via the A-domain and domains with desired binding specificity can be selected, for example, by phage display techniques. The target specificity of the different A-domains contained in an avimer may, but do not have to be identical (Weidle UH, et al., (2013), Cancer Genomics Proteomics; 10(4):155-68).

"Nanofitins" and also an "Affitins" are antibody mimetic proteins that are derived from the DNA binding protein Sac7d of Sulfolobus acidocaldarius. Nanofitins and Affitins usually have a molecular weight of around 7kDa and are designed to specifically bind a target molecule by randomising the amino acids on the binding surface (Mouratou B, Béhar G, Paillard-Laurance L, Colinet S, Pecorari F., (2012) Methods Mol Biol.; 805:315-31 and Koide et al. 1998, J. Mol. Biol. 284:1141-51).

The term "Affilin", as used herein, refers to antibody mimetics that are developed by using either gamma-B crystalline or ubiquitin as a scaffold and modifying amino-acids on the surface of these proteins by random mutagenesis. Selection of affilins with the desired target specificity is effected, for example, by phage display or ribosome display techniques. Depending on the scaffold, affilins have a molecular weight of approximately 10 or 20kDa. As used herein, the term affilin also refers to di- or multimerised forms of affilins (Weidle UH, et al., (2013), Cancer Genomics Proteomics; 10(4):155-68).

As used herein, the term "β-Wrapins" designates affibody protein homodimers with a disulfide bond between the pair of Cys28 residues connecting the two identical monomer subunits, referred to as subunits 1 and 2. The scaffold used in engineering β-wrapins is ZAβ₃, an Aβ-binding affibody protein that not only prohibits the initial aggregation of Aβ monomers into toxic forms, but also dissociates preformed oligomeric aggregates by sequestering and stabilizing a β-hairpin conformation of Aβ monomers (Orr et al. (2018), Computers & Chemical Engineering, 116(4):322-332).

As used herein, the term "ABD-Derived Affinity Proteins (ADAPT)" refers to a class of antibody mimetics that has been created using the albumin-binding domain (ABD) of streptococcal protein G as a stable protein scaffold (Garousi et al (2015), Cancer Res.; 75(20):4364-71). By diversifying a surface of the domain that is not directly involved in albumin binding, molecules can be selected to bind a novel target and still retain their ability to bind albumin. This strategy has been used to select binders to a number of proteins, for example, the cancer-related epidermal growth factor receptor 3.

As used herein "Raslns" are 10Fnlll-based antibody mimetics. Hence, they use the 10th domain of human fibronectin as their scaffold Raslns are disulfide-free intrabodies. They were shown to be stable inside cells and also when fused with a fluorescent protein label (Cetin et al. (2017), J Mol Biol.; 429(4):562-573).

As used herein, the term "FingRs (Fibronectin intrabodies generated with mRNA display)" designates recombinant antibody-like proteins also being based on the 10Fnlll scaffold (Gross et al. (2013), Neuron.; 78(6): 971-985.).

As used herein, the term "Pronectins" designates recombinant antibody-like proteins being based on the fourteenth type-III scaffold of human fibronectin (14Fn3). The well-characterized fibronectin protein is prevalent throughout the human body. Human fibronectin, an extracellular protein, is naturally abundant in human serum. Intelligent loop-diversity has been designed to closely mimic the natural human repertoire and avoid sequence immunogenicity. The intrinsic properties of a Pronectin align with the pharmacological properties needed to make it a successful drug, including high potency, specificity, stability, favorable small size, and high-yield production in *E*. *coli* and yeast (http://www.protelica.com/pronectin_tech.html).

As used herein, the term "Centyrins" designates recombinant antibody-like proteins being based on the consensus tenascin FN3 framework (Tencon) (Diem et al. (2014), Protein Eng., Des. and Sel. 27, 419-429). Centryins against different targets, e.g. human c-MET, rTNFα and mIL-17A, were generated.

As used herein, "Affimers" refer to small proteins that bind to target molecules with similar specificity and affinity to that of antibodies. These engineered non-antibody binding proteins are designed to mimic the molecular recognition characteristics of monoclonal antibodies in different applications. In addition, these affinity reagents have been optimized to increase their stability, make them tolerant to a range of temperatures and pH, reduce their size, and to increase their expression in *E*. *coli* and mammalian cells. Derived from the cysteine protease inhibitor family of cystatins, which function in nature as cysteine protease inhibitors, these 12-14 kDa proteins share the common tertiary structure of an α-helix lying on top of an anti-parallel β-sheet (Tiede et al. (2017), eLife.; 6: e24903).

The class of recombinant antibody-like proteins designated as "Adhirons" herein is based on a phytocystatin consensus sequence as the scaffold (Tiede et al. (2014) Protein Eng. Des. Sel. 27, 145-55).

The class of recombinant antibody-like proteins designated as "αRep" herein is derived from alpha-helicoidal HEAT-like repeat protein scaffolds. In more detail, The αRep proteins are derived from a natural family of modular proteins comprising alpha-helical repeats, related to HEAT repeats, named after Huntingtin, the elongation factor 3 (EF3), the protein phosphatase 2A (PP2A), and the yeast kinase TOR. The association of several HEAT repeats forms alpha-solenoids of various lengths, which are naturally found in a number of cellular proteins involved in intracellular transport and protein-protein interaction (Hadpech et al. (2017), Scientific Reports; 7:Article number16335).

As used herein, the term "Repebodies" designates recombinant antibody-like proteins which are composed of leucine-rich repeat (LRR) modules. In more detail, the binding scaffold of Repebodies is based on variable lymphocyte receptors, which are nonimmunoglobulin antibodies composed of LRR modules in jawless vertebrates. A template scaffold was first constructed by joining consensus repeat modules between the N- and C-capping motifs of variable lymphocyte receptors. The N-terminal domain of the template scaffold was redesigned based on the internalin-B cap by analyzing the modular similarity between the respective repeat units using a computational approach (Lee at al. (2012), Proc Natl Acad Sci; 109(9): 3299-3304).

As used herein, the term "i-bodies" refers to recombinant antibody-like proteins built on the scaffold of a human protein and engineered with two loops that mimic the shape of shark antibodies. These loops are responsible for binding or interacting with a particular target (in circulation or on a cell) that is causing disease. The i-body is a human analogue of the antigen binding domain of the shark antibody, which combines the advantages of monoclonal antibodies (high target specificity and affinity) with the beneficial stability features of small molecules (https://www.ibodies.eu/).

As used herein, the term "Fynomer" refers to a non-immunoglobulin-derived binding polypeptide derived from the human Fyn SH3 domain. Fyn SH3-derived polypeptides are well-known in the art and have been described e.g. in Grabulovski et al. (2007) JBC, 282, p. 3196-3204, WO 2008/022759, Bertschinger et al (2007) Protein Eng Des Sel 20(2):57-68, Gebauer and Skerra (2009) Curr Opinion in Chemical Biology 13:245-255, or Schlatter et al. (2012), MAbs 4:4, 1-12).

A "Kunitz domain peptide" is derived from the Kunitz domain of a Kunitz-type protease inhibitor such as bovine pancreatic trypsin inhibitor (BPTI), amyloid precursor protein (APP) or tissue factor pathway inhibitor (TFPI). Kunitz domains have a molecular weight of approximately 6kDa and domains with the required target specificity can be selected by display techniques such as phage display (Weidle et al., (2013), Cancer Genomics Proteomics; 10(4):155-68).

Cytokines, toxic compounds, and chemokines are of interest as fusion partners since they may be useful in the treatment or prevention of a disease, including cancer and immunological disorders. Fluorescent dyes and photosensitizers are useful as diagnostic compounds. Enzymes act as biological catalysts and accelerate chemical reactions. Depending on the nature of the enzyme it can be can of therapeutic or diagnostic value. Also depending on the nature of the radionuclide, the radionuclide can be of therapeutic (e.g. 177Lu) or diagnostic value (68Ga).

A nanoparticle is usually defined as a particle of matter that is between 1 and 100 nanometres (nm) in diameter. Peptide-nanoparticle conjugates (PNCs) have recently emerged as a versatile tool for biomedical applications. Synergism between the two promising classes of materials allows enhanced control over their biological behaviors, overcoming intrinsic limitations of the individual materials (Jeong et al. (2018), Nano Converg. 5:38 and Spicer et al. (2018), Chem Soc Rev.; 47(10):3574-3620). While the inhibitor of the first or second aspect of the invention is not a peptide in view of the presence of the Michael acceptor the inhibitor can be conjugated through the amino acids of the inhibitor to a nanoparticle in essentially the same manner as a peptide. Non-limiting examples of types of nanoparticles (NPs) are iron oxide NPs (e.g. iron oxide magnetic NPs or Amino-dextran-functionalized superparamagnetic iron oxide nanoparticles (SPION)), magnetic NPS (e.g. magnetic nanovesicles), metal NPs (e,g, gold or silver NPs), superparamagnetic spherical polymer NPs (e.g. Dynabeads or Superparamagnetic polymeric micelles (SPPM)), semi-conducting NPs, mesoporous silica NP, liposomes and polymer NPs (e.g. PEG-PLL-PLGA, Poly-lactic acid, PLG/ chitosan, PLA/chitosan or polyacryl NPs).

The present invention relates in a fourth aspect to a pharmaceutical or diagnostic composition comprising the cathepsin inhibitor of the first or second aspect and/or the fusion construct of the third aspect.

The definitions and preferred embodiments of the first to third aspect of the invention apply *mutatis mutandis* to the fourth aspect of the invention as far as being amenable with this fourth aspect.

In accordance with the present invention, the terms "pharmaceutical composition" and "diagnostic composition" relate to a composition for administration to a patient, preferably a human patient. As discussed above, a diagnostic composition may also be used in vitro. The pharmaceutical composition or diagnostic composition of the invention comprises at least one inhibitor of the first or second aspect and/or the fusion construct of the invention. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The preferred form is a solution. The concentration of the inhibitor in the solution is preferably about 20 mg/ml or about 130 µM, wherein the term about is preferably ±20%, more preferably ±10%.

These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g inhibitor per day. However, a more preferred dosage might be in the range of about 0.01 mg to about 100 mg, even more preferably about 0.1 mg to about 50 mg and most preferably about 0.5 mg to about 15 mg inhibitor per day.

Furthermore, the total pharmaceutically effective amount of the inhibitor of the first or second aspect in the pharmaceutical composition administered will typically in the range of about 10 to about 150 µg per kg of body weight, such as for example about 150, 140, 130, 120, 110, 100, 90, 80, 70, 60, 50, 40, 30, 20, or 10 µg per kg of body weight. More preferably, the pharmaceutically effective amount of the inhibitor in the pharmaceutical composition will be in the range of about 6.66 to about 100 nmol of the inhibitor per kg of body weight, such as for example about 100, 90, 80, 70, 60, 50, 40, 30, 20, 10, 9, 8, 7 or 6.66 nmol of the inhibitor per kg of body weight.

For systemic administration, a therapeutically effective amount or dose can be estimated initially from *in vitro* or ex *vivo* methods. For example, a dose can be formulated in animal models to achieve a circulating concentration range that includes the IC50 as determined in cell culture. Such information can be used to more accurately determine useful doses in humans. Initial doses can also be estimated from *in vivo* data, e.g. animal models, using techniques that are well known in the art. One of ordinary skill in the art can readily optimize administration to humans based on animal data and will, of course, rely on the subject being treated, on the subject's weight, the severity of the disorder, the manner of administration.

The length of treatment needed to observe changes and the interval following treatment for responses to occur vary depending on the desired effect. The particular amounts may be determined by conventional tests which are well known to the person skilled in the art.

Pharmaceutical compositions of the invention preferably comprise a pharmaceutically acceptable carrier or excipient. By "pharmaceutically acceptable carrier or excipient" is meant a non-toxic solid, semisolid or liquid filler, diluent, encapsulating material or formulation auxiliary of any type (see also Handbook of Pharmaceutical Excipients 6ed. 2010, Published by the Pharmaceutical Press). Examples of suitable pharmaceutical carriers and excipients are well known in the art and include phosphate buffered saline solutions, water, emulsions, such as oil/water emulsions, various types of wetting agents, sterile solutions, organic solvents including DMSO etc. Compositions comprising such carriers or excipients can be formulated by well-known conventional methods. The pharmaceutical composition may be administered, for example, parenterally, such as subcutaneously, intravenously, intramuscularly, intraperitoneally, transdermally, locally or topically to the skin. The pharmaceutical composition is preferably administered parenterally, more preferably subcutaneously, and most preferably via subcutaneous injection.

The diagnostic composition of the invention is, for example, useful in the detection of an undesired physiological cathepsin levels, e.g. in different cells, tissues or another suitable sample, comprising contacting a sample with the inhibitor of the first or second aspect of the invention or the fusion construct of the invention and detecting the presence of cathepsin in the sample. Accordingly, the diagnostic composition of the invention may be used for assessing the onset or status of a disease. As defined herein below, in particular cancers and immunological disorders can be diagnosed with the help of inhibitor of the first or second aspect of the invention or the fusion construct of the invention. In this context the fusion partner of the first or second aspect of the inhibitor of the invention is preferably a fluorescent dye, a photosentisizer, a radionuclide, or a contrast agent for medical imaging. Such fusion constructs are particularly suitable for diagnostic applications.

The diagnostic composition of the invention can be administered as sole active agent or can be administered in combination with other agents, if the diagnostic composition is, for example, used to identify sites of undesired physiological cathepsin levels within a subject. In general terms the diagnostic composition of the invention is used in the diagnosis of a cathepsin-mediated disease or a cathepsin - mediated organ damage, such as cancers and immunological disorders.

The present invention relates in a fifth aspect to the cathepsin inhibitor of the first or second aspect, the fusion construct of the third aspect or the pharmaceutical of the fourth aspect for use in treating cancer or an immunological disorder, wherein the cancer is preferably selected from the group consisting of breast cancer, ovarian cancer, endometrial cancer, vaginal cancer, vulvacancer, bladder cancer, salivary gland cancer, pancreatic cancer, thyroid cancer, kidney cancer, lung cancer, cancer concerning the upper gastrointestinal tract, colon cancer, colorectal cancer, prostate cancer, squamous-cell carcinoma of the head and neck, cervical cancer, glioblastomas, malignant ascites, myeloma, lymphomas and leukemias.

Cathepsins are highly expressed in various human cancers, associated with tumor metastasis (Tan et al. (2013), World J Biol Chem.; 4(4):91-101). As a group of lysosomal proteinases or endopeptidases, each member has a different function, playing different roles in distinct tumorigenic processes such as proliferation, angiogenesis, metastasis, and invasion. The particular role of cathepsin K and L in cancer has been discussed herein above. The cathepsin inhibitors of the invention are suitable forthe treatment of cancer.

Cathepsins are also involved in producing immune modulators by the limited proteolysis processing. For example, cathepsin K (CatK) is involved in toll-like receptor 9 (TLR9) activation. Cathepsin L plays a role in the immune system by degrading the li chain in MHC class II processing, which is a critical step in antigen presentation. The expression of cathepsin L in the thymus was shown to be essential for the development of natural killer cells.

Immunological disorders are diseases or conditions caused by a dysfunction of the immune system. The expression "immunological disorder" in the context of the present invention includes autoimmune disorders that involve activity of antigen presenting cells as B-cell, macrophages, NK20 cell and T-cells. Non-limiting examples of immunological disorders are periodontitis, diabetes, cardiovascular diseases, and neurodegenerative disease, including Alzheimer disease, systemic lupus erythematosus, Sjogren's syndrome, coeliac disease and psoriasis.

In accordance with preferred embodiment of the invention treating cancer is an anti-cancer immunotherapy or an anti-metastatic therapy.

The above preferred specific cancer types are non-limiting but preferred types of cancer that can be treated with the inhibitor, the fusion protein or the composition of the invention. The list comprises the most frequent types of cancer in human, noting that lung, breast and colorectal cancer are the 3 most abundant types of cancer.

The definitions and preferred embodiments of the first to fourth aspect of the invention apply *mutatis mutandis* to the fifth aspect of the invention as far as being amenable with this fifth aspect.

Unless otherwise defined, all technical and scientific terms used herein have the same meaning as commonly understood by one of ordinary skill in the art to which this invention belongs. In case of conflict, the patent specification including definitions, will prevail.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

This also holds true for alternatives in different claims that depend from each other. Thus, if claim 1 recites three alternatives of the same category and claim 2 recites three alternatives of a different category as recited in claim 1, and refers back to claim 1, all combinations of the alternatives as recited in claims 1 and 2 are explicitly disclosed herein.

The above considerations apply *mutatis mutandis* to all appended claims.

The figures show.

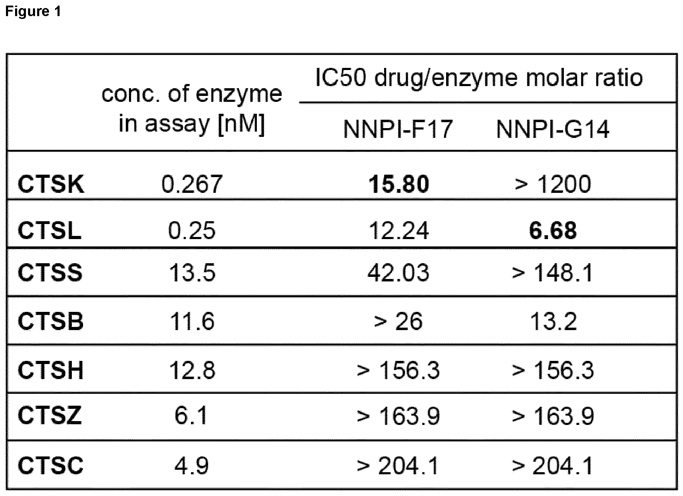

**Figure 1****.** IC50 values illustrating potency and specificity of NNPI- F17 and NNPI-G14 based on FRET assays performed on a panel of 7 different cysteine cathepsin proteases (n=3 for each condition in each FRET assay). IC50 values are given as a ratio of drug/enzyme molar concentration in the assay.
**Figure 2****.** a) Single-site mutagenesis screening library applied to CTSK and CTSL. b-c) Site mutagenesis screening results after testing inhibition potency on CTSK (b) and CTSL (c) (n=4). d) Sequence and inhibitory potency of different NNPIs with single amino acid substitutions tested on CTSK compared to NNPI-E15 (n=3). e) Sequence and inhibitory potency of different NNPIs with alternative single amino acid substitutions tested on CTSK compared to the most potent NNPIs identified in the previous screening step (labeled in dark blue). The best NNPIs identified are labeled in light blue (n=3). f) Sequence and inhibitory potency of different NNPIs combining up to 3 amino acid changes compared to NNPI-E15 (n=3), tested on CTSK. The most potent NNPI is labeled in red. g) Dose-response curve quantifying the inhibitory potency in FRET assays of NNPI-F17 on CTSK (n=3). A schematic representation of the structure of the NNPI is given to the left. h) Sequence and inhibitory potency of different NNPIs with single amino acid substitutions tested on CTSL compared to NNPI-E15 (n=3). i) Sequence and inhibitory potency of different NNPIs with alternative single amino acid substitutions tested on CTSL compared to the most potent NNPIs identified in the previous screening step (labeled in dark blue). The best NNPls identified are labeled in light blue (n=3). j) Sequence and inhibitory potency of different NNPIs combining up to 3 amino acid changes compared to NNPI-E15 (n=3), tested on CTSL. The most potent NNPIs are labeled in red. k) Dose-response curve quantifying the inhibitory potency in FRET assays of NNPI-G14 on CTSL (n=3). A schematic representation of the structure of the NNPI is given to the left.

The examples illustrate the invention.

### Example 1 - Results

Cysteine cathepsins are promising therapeutic targets in the oncological field as well as for the therapy of osteoporosis and autoimmune diseases. To develop covalent inhibitors that would block the enzymatic activity of cysteine cathepsins, non-natural peptide inhibitors (NNPI) functionalized to react with the catalytic cysteine and form a covalent bond were designed. The NNPIs are composed of a 7 amino acids sequence integrated with a Michael acceptor in the peptide backbone (between the 4th and the 5th amino acid). Thus, the Michael acceptor acts as a reactive warhead capable of forming a covalent bond with the cysteine in the active site of the cathepsin target.

While testing the selectivity of different prototype NNPIs on various cathepsins, it was noticed that NNPI-E15 was inhibiting CTSK and CTSL better than other cathepsins. Therefore, the sequence of NNPI-E15 was used as a starting point to create an NNPI library (n = 32 NNPIs) to determine the impact of each amino acid type (i.e. small, apolar, polar, aromatic, negatively charged or positively charged) in different positions when inhibiting CTSK and CTSL (Fig. 2a). In the screening 4 promising amino acid changes for CTSK (V5W, M6L, M6W, and W7R) and 4 for CTSL (R3L, L4W, M6L and W7T) were identified and confirmed (Fig. 2b-d,h).

Next, alternative changes in positions 3, 4, 5, 6 and 7 were tested, and it was noticed that V5Y and M6I improved potency on CTSK while L4Y, V5H and M6I were better changes for CTSL inhibition (Fig. 2e,i). In the last step the best mutations were combined, although L4W was selected instead of L4Y for the CTSL inhibitor, as a tyrosine in position 4 would increase the affinity for CTSB, and M6L was preferred to M6I, as the latter also improved inhibition of CTSK (Fig. 2e). Interestingly, for CTSK, the combination yielding the strongest inhibition was not the one in which all promising amino acid changes were implemented, but two changes in position 6 and 7 were sufficient to identify NNPI-F17 as the best CTSK inhibitor (IC50 = 2.7 nM) (Fig. 2f-g), suggesting that the effects of these changes are not purely additive. For CTSL the combination of a tryptophan in position 4, a leucine in position 3 and a methionine in position 6 in NNPI-G12 yielded to the most potent inhibitor (Fig. 2j). However, given its poor solubility and the similar potency of NNPI-G14, the latter was selected as the best CTSL inhibitor (IC50 = 1.7 nM) (Fig. 2j-k).

Then, NNPI-F17 and NNPI-G14 were profiled against five additional cathepsins and confirmed the good selectivity of these two inhibitors (Fig. 1).

In summary, by using an initial rational design and then a screening approach, several potent and two very potent NNPIs inhibiting CTSK and CTSL were identified.

### Example 2 - Material & Methods

### FRET assay: Cathepsin K (CTSK) and cathepsin L (CTSL)

Commercially available human CTSK (Sigma-Aldrich, cat #219461-25UG) and CTSL purified proteins (R&D systems, cat #952-CY-010) were activated and used for the assay following the protocol suggested by the manufacturer for CTSL. In brief, CTSK or CTSL was diluted to 20 µg/ml or 40 µg/ml respectively, in Assay Buffer (50 mM MES, 5 mM DTT, 1 mM EDTA, 0.005% (w/v) Brij-35, pH 6.0), and incubated on ice for 15 minutes. After activation, CTSK or CTSL was diluted to 0.02 µg/ml in Assay Buffer. The substrate peptide Z-Leu-Arg-AMC (R&D systems, cat #ES008) was then diluted to 20 µM in Assay buffer (40 µM for CTSL).

Next, 50 µl of the enzyme solution was loaded in a 96-well plate, inhibitors to be tested were added to the enzyme and incubated for 30 minutes, and then the reaction was started by adding 50 µl of the fluorogenic substrate. A substrate blank containing 50 µl of Assay Buffer and 50 µl of substrate without any cathepsin was included. Fluorescent emission was measured with SpectraMax iD3 (Molecular Devices) in kinetic mode for 60 minutes (excitation: 380 nm, emission: 460 nm).

### Single-site mutagenesis screening

A library of 32 NNPIs was screened by FRET assay on CTSK and CTSL by using 384-well plates. For each of the amino acid positions from 2 to 7 in the original NNPI-E15 structure, 6 different amino acid substitutions were tested (A, L, T, W, E or R). The final concentrations of enzyme and substrate were the same as the ones described above for the respective FRET assays. The final reaction volume per well was 20 µl. The inhibitor concentration was 30 nM. Fluorescent emission was measured with a BioTek Neo HTS plate reader.

## Claims

1. A cathepsin K inhibitor comprising or consisting of Formula (I)
(X₁)(X₂)(X₃)(X₄)(Y)(X₅)(X₆)(X₇) Formula (I)
wherein
(X₁) is the amino acid K;
(Xz) is an amino acid selected from R and W, and is preferably R;
(X₃) is an amino acid selected from R and W, and is preferably R;
(X₄) is the amino acid L;
(X₅) is an amino acid selected from Y, V and W, and is preferably V or Y, and is most preferably V;
(Xe) is an amino acid selected from I, M, L, Wand R, and is preferably I, L or W; and is most preferably I;
(X₇) is an amino acid selected from H, W, A T, R; and is preferably H or R, and is most preferably R; and
(Y) is a Michael acceptor.

2. The cathepsin K inhibitor of claim 1, wherein the cathepsin K inhibitor comprises or consists of KRRL(Y)VIR, KRRL(Y)VMR, KRRL(Y)VRW, KRRL(Y)VWW, KRRL(Y)VLW, KRRL(Y)WMW, KRRL(Y)VIW, KRRL(Y)YMW, KRRL(Y)VMH, KRRL(Y)VIH, KRRL(Y)YIR or KRRL(Y)YIH or KRRL(Y)YIW.

3. The cathepsin K inhibitor of claim 2, wherein the cathepsin K inhibitor comprises or consists of KRRL(Y)VIR, KRRL(Y)VIW, KRRL(Y)YMW, KRRL(Y)VMH, KRRL(Y)VIH,
KRRL(Y)YIR, KRRL(Y)YIH or KRRL(Y)YIW.

4. The cathepsin K inhibitor of claim 3, wherein the cathepsin K inhibitor comprises or consists of KRRL(Y)VIR.

5. A cathepsin L inhibitor comprising or consisting of Formula (II)
(X₁)(X₂)(X₃)(X₄)(Y)(X₅)(X₆)(X₇) Formula (II)
wherein
(X₁) is the amino acid K;
(X₂) is the amino acid R;
(X₃) is an amino acid selected from R, L and M, and is preferably L;
(X₄) is an amino acid selected from L, W, F and Y, and is preferably W;
(X₅) is an amino acid selected from V, H and R, and is preferably V;
(X₆) is an amino acid selected from M, L, I, Wand R, and is preferably L;
(X₇) is an amino acid selected from W, A T, R; and is preferably W; and
(Y) is a Michael acceptor.

6. The cathepsin L inhibitor of claim 5, wherein the cathepsin L inhibitor comprises or consists of KRLW(Y)VLW, KRRW(Y)VMW, KRRW(Y)VLW, KRLW(Y)VMW, KRRF(Y)VMW, or KRRY(Y)VMW.

7. The cathepsin L inhibitor of claim 6, wherein the cathepsin L inhibitor comprises or consists of KRLW(Y)VLW.

8. The cathepsin inhibitor of any one of claims 1 to 7, wherein the Michael acceptor is an enone, a nitro group or a sulfonyl fluoride and is preferably an enone.

9. The cathepsin inhibitor of claim 8, wherein the Michael acceptor has the general formula (III) or (IV) 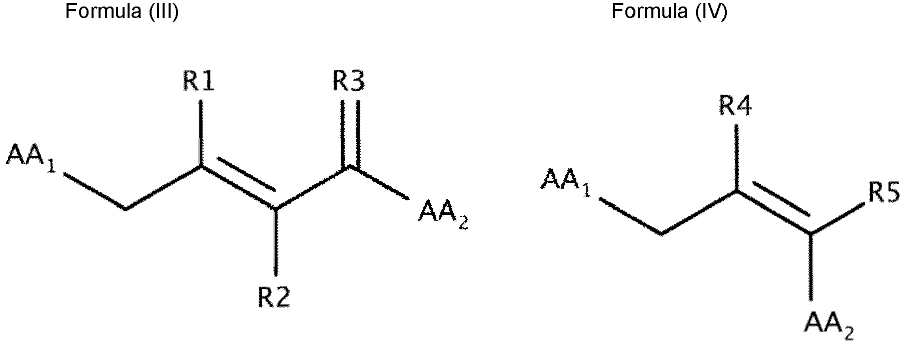 wherein
R1, R2 and R4 each independently is H or a halogen, wherein the halogen is preferably F;
R3 is chalcogen and is preferably O or S; and

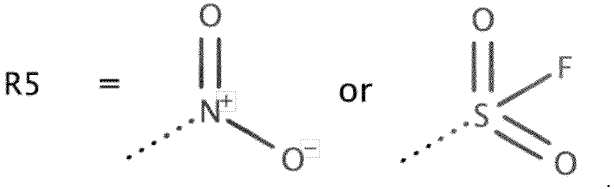

10. The cathepsin inhibitor of any one of claims 1 to 8, wherein the cathepsin inhibitor inhibits cathepsin K or L with IC₅₀ of below 100 nM, preferably below 50nM and most preferably below 10nM.

11. The cathepsin inhibitor of any one of claims 1 to 10, wherein the cathepsin inhibitor comprises at its N- or C-terminus, preferably at its C-terminus a linking moiety.

12. The cathepsin inhibitor of claim 11, wherein the linking moiety is an azide, alkyne phenol, secondary or tertiary amine, hydroxyl group, carbamate, or carbonate group, and is preferably an azide.

13. A fusion construct, wherein the cathepsin inhibitor of any one of claims 1 to 12 is fused to a heterologous compound, preferably fused to a pharmaceutically and/or diagnostically active compound.

14. The fusion construct of claim 13, wherein the heterologous compound is selected from the group consisting of:
(i) antibody or fragment thereof;
(ii) an antibody mimetic, preferably selected from the group consisting of Anticalins, Affibodies, Adnectins, DARPins, Avimers, Nanofitins, Affilins, β-Wrapins, ADAPT, Monobodies, Raslns, FingRs, Pronectins, Centyrins, Affimers, Adhirons, Affitins, αReps, Repebodies, i-bodies, Fynomers and Kunitz domain proteins;
(iii) a cytokine, preferably cytokines selected from the group consisting of IL-2, IL-12, TNF-alpha, IFN alpha, IFN beta, IFN gamma, IL-10, IL-15, IL-24, GM-CSF, IL-3, IL-4, IL-5, IL-6, IL-7, IL-9, IL-11, IL-13, LIF, CD80, B70, TNF beta, LT-beta, CD-40 ligand, Fas-ligand, TGF-beta, IL-1alpha and IL-1beta;
(iv) a toxic compound, preferably a small organic compound or polypeptide, and preferably a toxic compound selected from the group consisting of calicheamicin, neocarzinostatin, esperamicin, dynemicin, kedarcidin, maduropeptin, doxorubicin, daunorubicin, auristatin, Ricin-A chain, modeccin, truncated Pseudomonas exotoxin A, diphtheria toxin and recombinant gelonin;
(v) a chemokine, preferably a chemokine selected from the group consisting of IL-8, GRO alpha, GRO beta, GRO gamma, ENA-78, LDGF-PBP, GCP-2, PF4, Mig, IP-10, SDF-1alpha/beta, BUNZO/STRC33, I-TAC, BLC/BCA-1, MIP-1alpha, MIP-1 beta, MDC, TECK, TARC, RANTES, HCC-1, HCC-4, DC-CK1, MIP-3 alpha, MIP-3 beta, MCP-1-5, Eotaxin, Eotaxin-2, I-309, MPIF-1, 6Ckine, CTACK, MEC, Lymphotactin and Fractalkine;
(vi) a fluorescent dye, preferably a component selected from a Alexa Fluor or Cy dye;
(vii) a photosensitizer, preferably bis(triethanolamine)Sn(IV) chlorine6 (SnChe6);
(viii) a pro-coagulant factor, preferably a tissue factor;
(ix) an enzyme, preferably an enzyme selected from the group consisting of carboxy-peptidases, glucuronidases and glucosidases;
(x) a radionuclide selected either from the group of gamma-emitting isotopes, preferably 99mTc, 123I, 111In, or from the group of positron emitters, preferably 18F, 64Cu, 68Ga, 86Y, 124I, or from the group of beta-emitters, preferably 1311, 90Y, 177Lu, 67Cu, or from the group of alpha-emitters, preferably 213Bi, 211At;
(xi) nanoparticles.

15. A pharmaceutical or diagnostic composition comprising the cathepsin inhibitor of any one of claims 1 to 12 and/or the fusion construct of claim 13 or 14.
